Europäisches Patentamt

European Patent Office

Office européen des brevets

(11) Numéro de publication: **0 296 923 B1**

(12) **FASCICULE DE BREVET EUROPEEN**

(45) Date de publication de fascicule du brevet: **09.09.92**

(51) Int. Cl.5: **A61K 9/127**, A61K 7/00, A61K 35/78

(21) Numéro de dépôt: **88401420.0**

(22) Date de dépôt: **10.06.88**

(54) **Composition de liposomes contenant un extrait de mûrier.**

(30) Priorité: **12.06.87 FR 8708235**

(43) Date de publication de la demande:
**28.12.88 Bulletin 88/52**

(45) Mention de la délivrance du brevet:
**09.09.92 Bulletin 92/37**

(84) Etats contractants désignés:
**AT BE CH DE ES GB GR IT LI LU NL SE**

(56) Documents cités:
**WO-A-84/03836**
**FR-A- 2 582 941**

**CHEMICAL ABSTRACTS, vol.93, no.5, 04 août 1980, Columbus, OH (US); O.L.PODHAJCER et al., p.36, no.36862s**

**CHEMICAL ABSTRACTS, vol.98, no.9, 28 février 1983, Columbus, OH (US); S.YOSHIDA, no.69026d**

(73) Titulaire: **LVMH RECHERCHE**
**48-50, rue de Seine, B.P. 79**
**F-92703 Colombes Cédex(FR)**

(72) Inventeur: **Meybeck, Alain**
**Les poissons-Apt 242 20ter Rue de Bezons**
**F-92400 Courbevoie(FR)**
Inventeur: **Dumas, Marc**
**63, bd Gambetta**
**F-92700 Colombes(FR)**

(74) Mandataire: **Portal, Gérard et al**
**Cabinet Beau de Loménie 55, rue d'Amsterdam**
**F-75008 Paris(FR)**

Rank Xerox (UK) Business Services

## Description

La présente invention concerne essentiellement une composition à base de phases lamellaires lipidiques hydratées ou de liposomes contenant un extrait de mûrier, de préférence Morus Alba, et une composition pharmaceutique, notamment dermatologique, à activité dépigmentante, ou anti-inflammatoire, ou cosmétique,l'incorporant. On préfère selon l'invention un extrait obtenu à partir des parties aériennes ou souterraines du mûrier, de préférence Morus Alba L. ou Morus Rubra, notamment les feuilles, les extrêmités des tiges, l'écorce des rejets ou des racines.

On sait que le mûrier pousse partout naturellement en montagne et dans les champs. C'est une plante qui est cultivée depuis longtemps pour la sériculture. On a déjà utilisé depuis fort longtemps, notamment dans la médecine chinoise, les parties du mûrier, en particulier l'écorce de ses racines ou de ses rejets, ou des extraits de cette écorce encore dénommés "sohakuhi".

On attribue à l'écorce séchée des rejets ou des racines du mûrier ou à ses extraits des vertus thérapeutiques, notamment antiphlogistiques, diurétiques, fébrifuges, comme remède contre la douleur, dans le traitement de la bronchite, de l'asthme, etc.

Il a été découvert relativement récemment dans l'extrait d'écorce des rejets ou des racines de mûrier, ou sohakuhi, la présence de composés de blanchiment que l'on a incorporés dans les compositions cosmétiques en vue d'inhiber l'activité de la tyrosinase, qui est une enzyme responsable de la coloration de la peau, en obtenant ainsi une activité dépigmentante (voir C.A. Essential Oils, Cosmetics, volume 88, 1978, page 227, abrégé 88:65874z ou JP-A-50-135-236).

Pour des études pharmacologiques, voir le document Japan Journal of Pharmacology, 1976, 26 (4), 461-9 ou C.A. volume 85, 1976, page 48, abrégé 85:137465e. Une application d'extrait de mûrier comme agent antidiabétique est décrite dans FR-A-2 336 941.

On a par ailleurs déjà isolé de nombreuses substances à partir de l'extrait d'écorce de rejets ou de racines de mûrier, dont la grande majorité constitue des principes actifs. IL s'agit en général de flavones, de dérivés de celles-ci, et en particulier des kuwanones.

Le premier document est relatif à l'isolation d'une poudre cristalline blanche (C.A., 45 (1951), abrégé 9513g.

L'isolation d'une poudre blanche-jaunâtre est décrite dans C.A. - 57 (1962), abrégé 3970g.

L'isolation de 4 flavones est décrite pour la première fois dans Tetrahedron Letters N° 14, pages 1715-1719, 1968, avec un abrégé correspondant dans Chemical Abstracts, volume 69, 1968, page 3340, abrégé 35869s. D'autres flavones comme la rubraflavone A, B, C ou D sont décrites dans l'Indian Journal of Chemistry, volume 12, 2 Mai 1974, pages 431-436 avec un abrégé dans C.A., volume 82, 1975, page 557, abrégé 57520s. Du *-tocophérol a également été isolé de l'écorce des racines du mûrier (C.A., volume 82, 1975, page 213, abrégé 70272a). D'autres flavones, comme le mulberranol et un phénol comme l'alboctalol, ont encore été isolés (C.A., 86 (1977), page 551, abrégé 86:139896r). Par la suite, d'autres flavones comme la morusine, la cyclomorusine ont été isolées et décrites dans C.A., volume 87, 1977, page 566, abrégé 87:167917n.

L'isolation de la kuwanone E est encore décrite dans C.A., volume 89, 1978, abrégé 89:211925f.

L'isolation de kuwanone A, B, C et de l'oxydihydromorusine est encore décrite dans C.A., volume 89, 1978, page 429, abrégé 89:103271c.

On a également décrit la synthèse de la tétrahydrokuwanone C tétraméthyléther dans Heterocycles, volume 9, N° 10, 1978, pages 1355-1366.

Une description plus détaillée de l'isolation de la kuwanone E est encore décrite dans Heterocycles, volume 9, N° 9, de 1978.

L'isolation de constituants phénoliques est décrite dans C.A., volume 90, 1979, abrégé 90:83661y. La structure de la moracénine D, constituant une flavone particulière, est décrite, avec son procédé d'extraction et comme présentant une activité hypotensive, dans Heterocycles, volume 16, N° 6, 1981 et aussi dans C.A., volume 94, 1981, abrégé 94:117759m.

L'isolation du Mulberrofurane B est encore décrite dans C.A., volume 95, 1981, abrégé 95:147106j.

On peut ainsi constater que l'emploi d'extrait d'écorce de racine de mûrier comme produit pharmaceutique ou cosmétique est bien connu, ainsi que l'isolation d'un certain nombre de substances actives.

Pour un résumé détaillé des procédés d'extraction, on peut également se reporter à Heterocycles, volume 15, N° 2, 1981, pages 1531 à 1567.

Par ailleurs, le document C.A. Volume 93, 1980, abrégé 93:368620 décrit l'encapsulation dans des liposomes de quercétine pour des effets sur la synthèse de l'ADN, la production de lactate et pour augmenter le taux d'adénosine $3',5'$-monophosphate cyclique.

On a aussi utilisé des extraits de feuilles de mûrier (Morus Alba) pour la préparation de compositions cosmétiques en raison de propriétés eudermiques et eutrophiques (voir FR-A-2 582 941).

Comme produit ayant une activité dépigmentante importante, on peut citer l'association de principes actifs dite : "trio de Kligman" qui est basée sur une combinaison de vitamine A acide ou trétinoïne, d'hydroquinone et d'un corticoïde (Dexaméthasone). Cependant, l'utilisation d'un tel produit a été en pratique limitée en raison d'un caractère irritant élevé, causé par la présence de la vitamine A acide qui est bien connue pour son caractère irritant, et des effets indésirables dus à l'hypervitaminose A (voir "The Journal of Investigative Dermatology, volume 73, N° 5, partie I, pages 354-358, et en particulier l'avant-dernier paragraphe de la page 357, publié en 1979).

C'est ce qui explique l'intérêt des extraits de mûrier, notamment des extraits d'écorce de rejets ou de racines de mûrier, qui ne présentent pas les inconvénients précités mais leur activité dépigmentante est faible.

Par ailleurs, on connaît déjà l'emploi de phases lamellaires lipidiques hydratées ou de liposomes dans des compositions pharmaceutiques ou des compositions cosmétiques dans lesquelles on incorpore divers principes actifs (FR-A-2 540 381).

Il a maintenant été découvert, de manière tout à fait surprenante et inattendue, que l'extrait de mûrier, notamment de rejets ou de racines de mûrier, ou de toute substance active ayant pu être isolée d'un tel extrait de mûrier, comme une flavone, en particulier une kuwanone, avait une activité exacerbée, lorsque cet extrait ou cette substance était au moins en partie incorporé dans une phase lamellaire lipidique hydratée ou dans des liposomes. Ceci concerne toutes les activités connues pour les extraits de mûrier ou les substances isolées de tels extraits, en particulier les kuwanones. Une amélioration encore plus radicale de l'activité a été observée en ce qui concerne l'activité dépigmentante et l'activité anti-inflammatoire.

Un tel effet d'exacerbation de l'activité est également obtenu si l'on incorpore dans les phases lamellaires lipidiques hydratées ou dans les liposomes une combinaison des extraits de mûrier, ou d'une substance active isolée de tels extraits, avec de l'acide kojique ou ses dérivés, notamment ses sels ou esters.

Une amélioration appréciable de l'activité est encore observée en combinant les extraits de mûrier avec l'hydroquinone seule ou un mélange d'hydroquinone avec de l'acide kojique ou ses dérivés, notamment ses sels ou esters.

Ainsi, la présente invention a pour but de résoudre le nouveau problème technique consistant en la fourniture d'une nouvelle formulation d'extrait de mûrier, ou de toute substance active isolée à partir d'un tel extrait ou reconstituée par synthèse chimique, permettant de potentialiser leur efficacité pour permettre leur utilisation dans des compositions pharmaceutiques, notamment dermatologiques, à activité dépigmentante ou anti-inflammatoire, ou comme composition cosmétique.

La présente invention résout pour la première fois de manière satisfaisante ce nouveau problème technique.

Ainsi, selon un premier aspect, la présente invention fournit une composition à base de phase lamellaires lipidiques hydratées ou de liposomes, caractérisée en ce que lesdites phases lamellaires lipidiques hydratées ou lesdits liposomes contiennent au moins en partie un extrait de mûrier, notamment Morus Alba L. ou Morus Rubra, ou au moins une substance active isolée d'un tel extrait ou obtenue par synthèse chimique, en particulier une kuwanone.

Selon une caractéristique particulière de cette composition, celle-ci contient un extrait de feuilles, d'extrêmités de tiges, d'écorce de rejets ou d'écorce de racines de mûrier, obtenu par une extraction par solvant, de préférence choisi parmi le groupe consistant d'un solvant polaire, en particulier une solution alcoolique ou hydro-alcoolique ou éthérée ; d'un solvant organique apolaire comme le n-hexane, le benzène ; ou une combinaison des deux, avantageusement en effectuant en premier lieu une extraction avec un solvant organique apolaire suivie d'une extraction avec un solvant polaire.

Selon un mode de réalisation avantageux de l'invention, la composition précitée est caractérisée en ce que les phases lamellaires lipidiques hydratées précitées contiennent en outre au moins en partie de l'acide kojique ou ses dérivés, notamment ses sels ou esters.

Selon encore un autre mode de réalisation particulièrement avantageux de l'invention, les phases lamellaires lipidiques hydratées précitées contiennent au moins en partie de l'hydroquinone seule ou en mélange avec de l'acide kojique ou ses dérivés, notamment ses sels ou esters.

Selon une variante de réalisation de cette composition, celle-ci est caractérisée en ce que l'extrait précité seul ou en mélange avec l'hydroquinone et/ou l'acide kojique ou ses dérivés, notamment ses sels ou esters, est introduit dans la phase lipidique des phases lamellaires lipidiques hydratées ou des liposomes.

Selon une autre variante de réalisation de cette composition, celle-ci est caractérisée en ce que l'extrait précité seul ou en mélange avec l'hydroquinone et/ou l'acide kojique ou ses dérivés, notamment ses sels ou esters, est introduit dans la phase aqueuse des phases lamellaires lipidiques hydratées ou des liposomes.

Selon un deuxième aspect, la présente invention concerne aussi une composition pharmaceutique, notamment dermatologique, à activité dépigmentante ou anti-inflammatoire ; ou une composition cosmétique, caractérisée en ce qu'elle comprend une composition à base de phases lamellaires lipidiques hydratées ou de liposomes, telle que précédemment définie.

De préférence, la proportion en poids de l'extrait sec de mûrier ou de toute substance active obtenue à partir d'un tel extrait ou par synthèse chimique est comprise entre 0,005 et 1 % relativement au poids total de la composition ; encore de préférence est comprise entre 0,005 et 0,1 % et encore mieux entre 0,05 et 0,1 % relativement au poids total de la composition.

Une telle composition peut contenir au moins en partie dans les phases lamellaires lipidiques hydratées en outre de l'acide kojique ou ses dérivés, notamment ses sels ou esters, de préférence à une teneur comprise entre 0,5 et 4 % en poids par rapport au poids total de la composition, encore de préférence entre 0,5 et 2 %, et encore mieux de 1 % environ.

D'autre part, lorsque la composition contient de l'hydroquinone, celle-ci est incorporée au moins en partie dans les phases lamellaires lipidiques hydratées à une teneur de préférence comprise entre 0,5 et 6 % en poids par rapport au poids total de la composition, de préférence entre 0,5 et 4 % et encore mieux environ égale à 2 %.

De même, cette composition pharmaceutique, notamment dermatologique, ou cosmétique peut selon une première variante être caractérisée en ce que l'extrait précité seul ou en mélange avec de l'hydroquinone et/ou l'acide kojique ou ses dérivés, notamment ses sels ou esters, est introduit dans la phase lipidique des phases lamellaires lipidiques hydratées ou des liposomes, tandis que selon une autre variante l'extrait précité seul ou en mélange avec l'hydroquinone et/ou l'acide kojique ou ses dérivés, notamment ses sels ou esters, peut être introduit dans la phase aqueuse des phases lamellaires lipidiques hydratées ou des liposomes.

L'invention couvre aussi les procédés de préparation de la composition à base de phases lamellaires lipidiques hydratées ou de liposomes précités ou la composition pharmaceutique, notamment dermatologique, à activité dépigmentante ou anti-inflammatoire, ou cosmétique, caractérisés en ce qu'on incorpore au moins en partie dans lesdites phases lamellaires lipidiques hydratées ou lesdits liposomes, un extrait de mûrier, notamment Morus Alba L. ou Morus Rubra, ou au moins une substance active isolée d'un tel extrait ou obtenue par synthèse chimique, en particulier une kuwanone.

Dans la présente description et les revendications, le terme "lipidique" dans l'expression "phase lamellaire lipidique" couvre toutes les substances comprenant une chaîne carbonée dite grasse, généralement supérieure à 5 atomes de carbone.

Selon l'invention, on utilise des lipides amphiphiles, c'est-à-dire constitués de molécules possédant un groupe hydrophyle indifféremment ionique ou non ionique et un groupe lipophile, ces lipides amphiphiles étant susceptibles de former des phases lamellaires lipidiques en précense d'une phase aqueuse. En particulier, citons parmi ces lipides : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un cérébroside ou un stéarate de polyglycérol oxyéthyléné.

D'autres buts, caractéristiques et avantages de l'invention apparaîtront clairement à la lumière de la description explicative qui va suivre faite en référence à plusieurs exemples illustratifs qui ne sauraient donc en aucune façon limiter la portée de l'invention. Dans les exemples, les pourcentages sont donnés en poids sauf indication contraire.

EXEMPLE 1 de l'invention

A- Préparation d'une composition sous forme de poudre contenant un extrait sec d'écorce de racine de mûrier.

On utilise comme extrait d'écorce de rejets de mûrier, un extrait commercialisé par la Société japonaise Marusen Seiyaku Co. Limited, constitué ici du lot N° 412033, qui se présente sous forme d'un liquide transparent, avec une faible odeur caractéristique, une couleur légèrement brune-jaunâtre, un pH de 4,4, une densité de 0,951, une teneur en résidu après évaporation de 1,83 % en poids/volume, une absorption UV E 1 %, 1 cm = 0,61 à 280 nm, une teneur en métaux lourds au maximum de 10 ppm et une teneur en arsenic au maximum de 2 ppm, avec réponse positive au test d'identification de tripénnoîde.

On prend 40 ml de cet extrait de mûrier que l'on évapore à sec. Puis on dissout cet extrait sec dans 300 ml de dichlorométhane auquel on ajoute 20 g de lécithine de soja hydrogénée, éventuellement en présence d'un antioxydant lyophile, par exemple 0,6 g d'$\alpha$-tocophérol.

On atomise la solution obtenue à 65°C, comme décrit dans le document FR-A-2 521 565 de manière à produire environ 21 g d'une fine poudre.

B- Préparation d'une composition sous forme de suspension de liposomes avantageusement homogénéisée

On disperse 20 g de la poudre obtenue à l'étape A dans une solution aqueuse tamponnée à pH 7,5 environ pour aboutir à un volume final de 500 ml. Généralement, on utilise une solution contenant 0,8 % en NaCl et 1,5 % NaH$_2$PO$_4$, dite "tampon phosphate", additionnée d'un stabilisateur antioxydant tel que de l'acide ascorbique à la concentration de 0,05 %.

On obtient ainsi, après homogénéisation soit aux ultrasons, soit dans un homogénéisateur sous pression, par exemple selon le procédé décrit dans le document FR-A-2 534 487, une suspension de liposomes.

Si par exemple on réalise une homogénéisation par un traitement aux ultrasons pendant 10 minutes, on obtient une dimension de liposomes moyenne de l'ordre de 112 nanomètres.

On observera qu'on peut réaliser diverses dilutions en modifiant la quantité d'extraits ajoutés au départ ou en augmentant le volume de la solution de dispersion, ce qui constitue un procédé aisé de préparation de diverses concentrations en extrait.

En l'absence d'une dilution, on obtient après cette étape B 500 ml de suspension homogénéisée.

C- Préparation d'une composition de liposomes homogénéisés sous forme de gel

Cette suspension homogénéisée peut être gélifiée par mélange avec un gel, tel qu'un gel de polymère vinylique, en particulier commercialisé sous la dénomination commerciale Carbopol®940.

Ainsi pour préparer ce gel de façon classique, on disperse 25 g de Carbopol®940 dans 500 g d'eau en présence d'un conservateur et d'un agent chélatant habituel, puis après gonflement, on neutralise à pH 7,5 avec de la triéthanolamine. Aux 500 ml de suspension homogénéisée obtenus à l'étape B ci-dessus, on ajoute 500 ml dudit gel, pour obtenir un volume total de 1000 ml.

Dans cette composition gélifiée, la concentration en extrait sec d'écorces de racines de mûrier est d'environ 0,073 % et la concentration en lécithine est de 2 %.

EXEMPLE 2

Composition de liposomes contenant de l'extrait d'écorce de racine de mûrier et de l'acide kojique

On procède comme à l'exemple 1-A, si ce n'est que l'on ajoute 10 g d'acide kojique soit directement dans l'extrait, soit de préférence dans le dichlorométhane.

On obtient une poudre contenant de l'acide kojique avec l'extrait sec de mûrier.

Cette poudre peut être dispersée selon la procédure décrite à l'exemple 1-B, puis la solution homogénéisée obtenue peut être gélifiée selon la procédure de l'exemple 1-C.

EXEMPLE 3

Composition de liposomes contenant de l'extrait d'écorce de racine de mûrier et de l'hydroquinone

On procède comme à l'exemple 1-A, si ce n'est que l'on ajoute 20 g d'hydroquinone soit directement dans d'extrait, soit de préférence dans le dichlorométhane.

De même, la poudre obtenue contenant de l'hydroquinone avec l'extrait sec de mûrier peut être dispersée selon la procédure decrite à l'exemple 1-B, puis la solution homogénéisée obtenue peut être gélifiée selon la procédure décrite à l'exemple 1-C.

EXEMPLE 4

Composition de liposomes contenant de l'extrait d'écorce de racine de mûrier, de l'acide kojique et de l'hydroquinone

On procède comme à l'exemple 1-A, si ce n'est que l'on ajoute 10 g d'acide kojique et 20 g d'hydroquinone, soit dans l'extrait, soit de préférence dans le dichlorométhane.

En outre, selon un mode de réalisation préféré, on ajoute à la solution aqueuse tamponnée, dans le cas du procédé d'atomisation, 4 g de sulfite de sodium et 5 g d'acide L-ascorbique, comme antioxydants.

EXEMPLE 5

Gel de liposomes contenant un extrait d'écorce de mûrier

Au lieu d'incorporer les principes actifs du mûrier dans la phase phospholipidique, on les introduit dans la phase aqueuse de la manière suivante :

On dissout 8 ml d'un extrait d'écorce de rejets de mûrier contenant environ 1 % de résidu sec dans 100 ml de tampon phosphate. On ajoute à cette solution 2 g d'un mélange intime obtenu par atomisation (suivant FR-A-2 521 565) de lécithine et sitostérol (9 : 1). On traite cette solution aux ultrasons jusqu'à obtenir une taille de liposomes inférieure à 150 nm. On rajoute 100 g d'un gel aqueux de Carbopol®940.

EXEMPLE 6

Utilisation de compositions conformes aux exemples 1 à 3 pour constituer une composition pharmaceutique ou cosmétique

On vérifie l'utilisation de la composition de l'exemple 1 à titre de composition pharmaceutique, notamment dermatologique, ou cosmétique en effectuant les expérimentations in vivo suivantes.

Mise en évidence de l'activité dépigmentante

Pour étudier l'activité dépigmentante des compositions selon l'invention, on utilise soit des souris Hairless à queue pigmentée, obtenues à 90 % dans la portée résultant de deux croisements successifs dont le premier croisement est réalisé à partir d'une souris C 57 noire femelle avec une souris mâle BL6-HRO ou eb-HRO, disponibles au centre de sélection et d'élevage d'animaux de laboratoire, en abrégé CSEAL du CNRS d'Orléans dont les mâles de la portée sont ensuite croisés à nouveau avec une souris femelle C57 noire.

On peut également utiliser des souris Hairless à oreilles pigmentées et yeux noirs Skh : HR-2 disponibles auprès du Temple University of Health Sciences Center Central Animal Facility de Philadelphie.

Pour réaliser les expérimentations, on applique sur une zone pigmentée le produit obtenu sous forme de gel à l'exemple 1 à raison de 5 jours par semaine pendant 6 semaines, et ce sur un lot de dix souris.

Ensuite, on prélève sur chaque souris, par biopsie, des fragments de peau de queue ou d'oreilles dans les zones où le produit a été appliqué que l'on met dans une solution de bromure de sodium pendant 2 heures, pour en séparer l'épiderme.

On dispose des échantillons de ces épidermes entre deux lames de quartz. On effectue un séchage puis une pesée.

Ensuite, on soumet ces fragments d'épidermes séchés et posée à une digestion par la trypsine à 37°C pendant 48 heures.

On effectue ensuite une filtration et une centrifugation à 3000 tr/min pendant 30 minutes.

On récupère le culot mélanique de chaque souris que l'on met en suspension dans de l'eau distillée en quantité nécessaire pour permetre la mesure de la densité optique. On obtient ainsi dix échantillons distincts.

On mesure la densité optique de ces 10 échantillons de manière classique à 400 nm.

Les valeurs relevées pour ces 10 échantillons sont ensuite ramenées à 100 mg de poids d'épiderme prélevé et on détermine une valeur moyenne de densité optique qui est seule prise en compte et est répertoriée au tableau I ci-après.

On a également effectué des essais comparatifs avec un gel contenant 0,073 % en poids d'extrait sec d'écorce de racine de mûrier provenant du même lot que celui utilisé à l'exemple 1 (composition $A_1$).

On a préparé encore une autre composition comparative ne contenant que le gel témoin (composition $A_2$).

Naturellement, le gel utilisé pour les compositions de comparaison est identique à celui utilisé pour préparer la composition de l'exemple 1.

Une autre composition comparative (composition $A_3$) utilise le "trio de Kligman" qui, comme mentionné précédemment, utilise la vitamine A acide et un corticoïde anti-inflammatoire (dexaméthasone).

6

On détermine également l'activité dépigmentante par comparaison avec les résultats obtenus à l'aide du trio de Kligman selon la formule suivant :

$$\text{Activité dépigmentante} = \frac{DO_T - DOA}{DO_T - DO_{T.K.}} \times 100$$

dans laquelle

$DO_A$ = densité optique obtenue avec la composition active testée (Invention Ex. 1 ou de comparaison $A_1$, $A_2$ ou $A_3$)

$DO_T$ = densité optique obtenue avec la composition témoin $A_2$

$DO_{T.K.}$ = densité optique obtenue avec la composition $A_3$ du trio de Kligman considéré comme témoin positif.

On peut observer à partir du tableau de résultats d'essais que l'emploi de vitamine A acide procure la meilleure efficacité dépigmentante.

Par ailleurs, un gel de 0,073 % d'extrait sec de mûrier (composition $A_1$) n'améliore pas l'activité dépigmentante par rapport à la composition témoin ($A_3$).

Par contre, un effet de synergie peut être observé par l'incorporation de l'extrait d'écorce de racine de mûrier dans des liposomes (valeur 356 par rapport à 509 pour le témoin et 530 pour le gel du même extrait sans liposomes).

On passe en effet d'une activité inexistante pour la composition $A_1$ par rapport à la composition de référence $A_3$ à une activité d'environ 37 % pour la composition de l'exemple 1, selon l'invention, par rapport à cette même composition de référence $A_3$.

Par ailleurs, les compositions selon l'invention ne présentent pas d'effets secondaires et surtout ne présentent pas l'effet irritant de la vitamine A acide qui a toujours constitué une limitation importante à l'utilisation du "trio de Kligman".

Ainsi, la composition selon l'invention se révèle très avantageuse comme substitut au trio de Kligman qui présente les inconvénients majeurs précités.

On donne ci-après divers exemples de compositions dermatologiques, dermo-cosmétiques.

EXEMPLE 7

Composition cosmétique anti-taches pour le visage, selon l'invention

Cette composition est constituée par le gel obtenu à l'état brut à l'exemple 1-C, que l'on applique matin et soir sur les taches mélaniques du visage jusqu'à atténuation des taches.

EXEMPLE 8

Crème pour les mains anti-taches

On prépare une crème par simple mélange des composants suivants, dans les proportions suivantes indiquées en grammes :

```
- poudre obtenue à l'exemple 1-A                    1 g
(ayant un rapport lécithine : extrait sec
de mûrier ( 9,65 : 0,35 )
- tampon phosphate                                 29 g
- émulsion W/O stabilisée                          70 g
                                                  ─────
                                                  100 g
```

EXEMPLE 9

Composition dermo-cosmétique anti-taches selon l'invention

On utilise les composants dans les proportions suivantes :

```
- poudre obtenue à l'exemple 2                          3 g
  (qui contient de la lécithine : extrait sec de
  mûrier = 9,65 : 0,35 ; avec acide kojique)
- tampon phosphate                                      47 g
- gel avec agent conservateur                           50 g
                                                       _____
                                                       100 g
```

Pour préparer la composition, on disperse tout d'abord la poudre dans le tampon phosphate, puis on ajoute le gel en obtenant ainsi une composition sous forme de gel, éventuellement avec chauffage selon les procédures décrites à l'exemple 1-B, 1-C.

EXEMPLE 10

Composition dermatologique liposomale anti-taches selon l'invention

On utilise les composants suivants dans les proportions suivantes en grammes :

```
- poudre obtenue à l'exemple 3                          4 g
  (qui contient de la lécithine : extrait sec
  de mûrier en rapport de 9,65 : 0,35, avec de
  l'hydroquinone)



- tampon phosphate                                      46 g
- gel avec agent conservateur                           50 g
                                                       _____
                                                       100 g
```

Pour obtenir cette composition, on procède comme à l'exemple 9.

Dans tous ces exemples 8 à 10, disperse la poudre qui contient éventuellement l'acide kojique ou hydroquinone dans le tampon phosphate selon la procédure décrite à l'exemple 1-B, puis on peut gélifier avec le gel selon la procédure décrite à l'exemple 1-C.

8

## TABLEAU I

### Résultats d'essais d'activité dépigmentante

| Composition | Ex 1 | $A_1$ | $A_2$ | $A_3$ |
|---|---|---|---|---|
| Densité optique pour 1000 mg de poids d'épiderme prélevée | 356 | 530 | 509 | 94 |
| Efficacité par rapport à $A_3$ (exprimée en %) | 37 | NS | 0 | 100 |

NS = non significatif

## Revendications
**Revendications pour les Etats contractants suivants : AT, BE, CH, DE, GB, IT, LI, LU, NL, SE**

1. Composition à base de phases lamellaires lipidiques hydratées ou de liposomes, caractérisée en ce que lesdites phases lamellaires lipidiques hydratées ou lesdits liposomes contiennent au moins en partie un extrait de mûrier, notamment Morus Alba L. ou Morus Rubra, ou au moins une substance active isolée d'un tel extrait ou obtenue par synthèse chimique, en particulier une kuwanone.

2. Composition selon la revendication 1, caractérisée en ce que l'extrait précité est un extrait de feuilles, d'extrêmités de tiges, d'écorce de rejets ou d'écorce de racines, obtenu par une extraction par solvant, de préférence choisi parmi le groupe consistant d'un solvant polaire, notamment une solution alcoolique ou hydro-alcoolique, ou une solution éthérée, d'un solvant organique apolaire, comme le n-hexane, le benzène, ou avantageusement une combinaison des deux.

3. Composition selon la revendication 1 ou 2, caractérisée en ce que les phases lamellaires lipidiques hydratées précitées contiennent en outre au moins en partie de l'acide kojique ou ses dérivés, notamment ses sels ou esters.

4. Composition selon la revendication 1, 2 ou 3, caractérisée en ce que les phases lamellaires lipidiques hydratées contiennent au moins en partie de l'hydroquinone seule ou en mélange avec de l'acide kojique ou ses dérivés, notamment ses sels ou esters.

5. Composition selon l'une des revendications 1 à 4, caractérisée en ce que l'extrait précité seul ou en mélange avec l'hydroquinone et/ou l'acide kojique ou ses dérivés, notamment ses sels ou esters, est introduit dans la phase lipidique des phases lamellaires lipidiques hydratées ou des liposomes.

6. Composition selon l'une des revendications 1 à 4, caractérisée en ce que l'extrait précité seul ou en mélange avec l'hydroquinone et/ou l'acide kojique ou ses dérivés, notamment ses sels ou esters, est

EP 0 296 923 B1

introduit dans la phase aqueuse des phases lamellaires lipidiques hydratées ou des liposomes.

7. Composition à activité dépigmentante ou anti-inflammatoire cosmétique ou pharmaceutique, notamment dermatologique, caractérisée en ce qu'elle comprend une composition à base de phases lamellaires lipidiques hydratées ou de liposomes contenant au moins en partie un extrait de mûrier, notamment Morus Alba L. ou Morus Rubra, ou au moins une substance active isolée d'un tel extrait ou obtenue par synthèse chimique, en particulier une kuwanone.

8. Composition cosmétique ou pharmaceutique, notamment dermatologique, selon la revendication 7, caractérisée en ce que l'extrait précité est un extrait de feuilles, d'extrêmités de tiges, d'écorce de rejets ou d'écorce de racines, obtenu par une extraction par solvant, de préférence choisi parmi le groupe consistant d'un solvant polaire, notamment une solution alcoolique ou hydro-alcoolique, ou une solution éthérée, d'un solvant organique apolaire, comme le n-hexane, le benzène, ou avantageusement une combinaison des deux.

9. Composition cosmétique ou pharmaceutique, notamment dermatologique, selon l'une des revendications 7 ou 8, caractérisée en ce que les phases lamellaires lipidiques hydratées précitées contiennent en outre au moins en partie de l'acide kojique ou ses dérivés, notamment ses sels ou esters.

10. Composition cosmétique ou pharmaceutique, notamment dermatologique, selon l'une des revendications 7 à 9, caractérisée en ce que les phases lamellaires lipidiques hydratées précitées contiennent au moins en partie de l'hydroquinone seule ou en mélange avec de l'acide kojique ou des dérivés, notamment ses sels ou esters.

11. Composition cosmétique ou pharmaceutique, notamment dermatologique, selon l'une des revendications 7 à 10, caractérisée en ce que l'extrait précité seul ou en mélange avec l'hydroquinone et/ou l'acide kojique ou ses dérivés, notamment ses sels ou esters, est introduit dans la phase lipidique des phases lamellaires lipidiques hydratées ou des liposomes.

12. Composition cosmétique ou pharmaceutique, notamment dermatologique,selon l'une des revendications 7 à 10, caractérisée en ce que l'extrait précité seul ou en mélange avec l'hydroquinone et/ou l'acide kojique et ses dérivés, notamment ses sels ou esters, est introduit dans la phase aqueuse des phases lamellaires lipidiques hydratées ou des liposomes.

13. Composition cosmétique ou pharmaceutique, notamment dermatologique, selon l'une des revendications 7 à 12, caractérisée en ce que la proportion en poids d'extrait de mûrier est comprise entre 0,005 et 1 %, de préférence 0,005 et 0,1 %, et encore de préférence entre 0,05 et 0,1 %, en poids par rapport au poids total de la composition.

14. Composition cosmétique ou pharmaceutique, notamment dermatologique, selon l'une des revendications 7 à 13, caractérisée en ce que l'acide kojique ou ses dérivés, notamment ses sels ou esters, constitue de 0,5 à 4 %, de préférence 0,5 à 2 %, et encore de préférence environ 1 %, en poids par rapport au poids total de la composition.

15. Composition cosmétique ou pharmaceutique, notamment dermatologique, selon l'une des revendications 7 à 14, caractérisée en ce que l'hydroquinone constitue de 0,5 à 6 %, de préférence de 0,5 à 4 %, encore de préférence environ 2 %, en poids par rapport au poids total de la composition.

16. Composition cosmétique, caractérisée en ce qu'elle comprend une composition à base de phases lamellaires lipidiques hydratées ou de liposomes contenant au moins en partie un extrait d'extrémités de tiges, d'écorce de rejets ou d'écorce de racines de mûrier, notamment Morus Alba L. ou Morus Rubra, ou au moins une substance active isolée d'un tel extrait ou obtenue par synthèse chimique, en particulier une kuwanone.

17. Composition selon la revendication 16, caractérisée en ce qu'elle est telle que définie à l'une des revendications 2 à 6.

18. Composition selon la revendication 16 ou 17, caractérisée en ce que la proportion respective en poids

10

d'extraits de mûrier, d'acide kojique ou ses dérivés ou d'hydroquinone est telle que définie à l'une des revendications 13, 14 ou 15.

**Revendications pour l'Etat contractant suivant : ES**

1.  Procédé de préparation d'une composition à base de phases lamellaires lipidiques hydratées ou de liposomes, caractérisé en ce qu'on incorpore au moins en partie dans lesdites phases lamellaires lipidiques hydratées ou lesdits liposomes, un extrait de mûrier, notamment Morus Alba L. ou Morus Rubra, ou au moins une substance active isolée d'un tel extrait ou obtenue par synthèse chimique, en particulier une kuwanone.

2.  Procédé de préparation selon la revendication 1, caractérisé en ce qu'on prépare un extrait de feuilles, d'extrêmités de tiges, d'écorce de rejets ou d'écorce de racines, obtenu par une extraction par solvant, de préférence choisi parmi le groupe consistant d'un solvant polaire, notamment une solution alcoolique ou hydro-alcoolique, ou une solution éthérée, d'un solvant organique apolaire, comme le n-hexane, le benzène, ou avantageusement une combinaison des deux.

3.  Procédé selon la revendication 1 ou 2, caractérisé en ce que les phases lamellaires lipidiques hydratées précitées contiennent en outre au moins en partie de l'acide kojique ou ses dérivés, notamment ses sels ou esters.

4.  Procédé selon la revendication 1, 2 ou 3, caractérisé en ce que les phases lamellaires lipidiques hydratées contiennent au moins en partie de l'hydroquinone seule ou en mélange avec de l'acide kojique ou ses dérivés, notamment ses sels ou esters.

5.  Procédé selon les revendications 1 à 4, caractérisé en ce qu'on introduit l'extrait précité seul ou en mélange avec l'hydroquinone et/ou l'acide kojique ou ses dérivés, notamment ses sels ou esters, dans la phase lipidique des phases lamellaires lipidiques hydratées ou des liposomes.

6.  Procédé selon l'une des revendications 1 à 4, caractérisé en ce qu'on introduit l'extrait précité seul ou en mélange avec l'hydroquinone et/ou l'acide kojique ou ses dérivés, notamment ses sels ou esters, dans la phase aqueuse des phases lamellaires lipidiques hydratées ou des liposomes.

7.  Procédé de préparation d'une composition à activité dépigmentante ou anti-inflammatoire cosmétique ou pharmaceutique, notamment dermatologique, caractérisé en ce qu'on incorpore au moins en partie dans lesdites phases lamellaires lipidiques hydratées ou lesdits liposomes, un extrait de mûrier, notamment Morus Alba L. ou Morus Rubra, ou au moins une substance active isolée d'un tel extrait ou obtenue par synthèse chimique, en particulier une kuwanone.

8.  Procédé selon la revendication 7, caractérisé en ce que l'extrait précité est un extrait de feuilles, d'extrêmités de tiges, d'écorce de rejets ou d'écorce de racines, obtenu par une extraction par solvant, de préférence choisi parmi le groupe consistant d'un solvant polaire, notamment une solution alcoolique ou hydro-alcoolique, ou une solution éthérée, d'un solvant organique apolaire, comme le n-hexane, le benzène, ou avantageusement une combinaison des deux.

9.  Procédé selon l'une des revendications 7 ou 8, caractérisé en ce que les phases lamellaires lipidiques hydratées précitées contiennent en outre au moins en partie de l'acide kojique ou ses dérivés, notamment ses sels ou esters.

10. Procédé selon l'une des revendications 7 à 9, caractérisé en ce que les phases lamellaires lipidiques hydratées précitées contiennent au moins en partie de l'hydroquinone seule ou en mélange avec de l'acide kojique ou des dérivés, notamment ses sels ou esters.

11. Procédé selon l'une des revendications 7 à 10, caractérisé en ce que l'extrait précité seul ou en mélange avec l'hydroquinone et/ou l'acide kojique ou ses dérivés, notamment ses sels ou esters, est introduit dans la phase lipidique des phases lamellaires lipidiques hydratées ou des liposomes.

12. Procédé selon l'une des revendications 7 à 10, caractérisé en ce qu'on introduit l'extrait précité seul ou

EP 0 296 923 B1

en mélange avec l'hydroquinone et/ou l'acide kojique et ses dérivés, notamment ses sels ou esters, dans la phase aqueuse des phases lamellaires lipidiques hydratées ou des liposomes.

13. Procédé selon l'une des revendications 7 à 12, caractérisé en ce qu'on incorpore de 0,005 et 1 %, de préférence 0,005 et 0,1 %, et encore de préférence entre 0,05 et 0,1 % en poids d'extrait de mûrier par rapport au poids total de la composition.

14. Procédé selon l'une quelconque des revendications 7 à 13, caractérisé en ce qu'on incorpore de 0,5 à 4 %, de préférence 0,5 à 2 % et encore de préférence environ 1 % en poids d'acide kojique ou ses dérivés, notamment ses sels ou esters par rapport au poids total de la composition.

15. Procédé selon l'une quelconque des revendications 7 à 14, caractérisé en ce qu'on incorpore de 0,5 à 6 %, de préférence de 0,5 à 4 % encore de préférence environ 2 % en poids d'hydroquinone par rapport au poids total de la composition.

16. Procédé de préparation d'une composition cosmétique à base de phases lamellaires lipidiques hydratées ou de liposomes, caractérisé en ce qu'on incorpore au moins en partie dans lesdites phases lamellaires lipidiques hydratées ou lesdits liposomes, un extrait d'extrémité de tige, d'écorce de rejets ou d'écorce de racines de mûrier, notamment Morus Alba L. ou Morus Rubra, ou au moins une substance active isolée d'un tel extrait ou obtenue par synthèse chimique, en particulier une kuwanone.

17. Procédé selon la revendication 16, caractérisé en ce que l'on procède comme décrit à l'une quelconque des revendications 2 à 6.

18. Procédé selon la revendication 16 ou 17, caractérisé en ce qu'on incorpore une concentration en poids respectivement d'extraits de mûrier, d'hydroquinone et/ou d'acide kojique ou ses dérivés telle que définie à l'une des revendications 13, 14 ou 15.

**Revendications pour l'Etat contractant suivant : GR**

1. Composition à base de phases lamellaires lipidiques hydratées ou de liposomes, caractérisée en ce que lesdites phases lamellaires lipidiques hydratées ou lesdits liposomes contiennent au moins en partie un extrait de mûrier, notamment Morus Alba L. ou Morus Rubra, ou au moins une substance active isolée d'un tel extrait ou obtenue par synthèse chimique, en particulier une kuwanone.

2. Composition selon la revendication 1, caractérisée en ce que l'extrait précité est un extrait de feuilles, d'extrêmités de tiges, d'écorce de rejets ou d'écorce de racines, obtenu par une extraction par solvant, de préférence choisi parmi le groupe consistant d'un solvant polaire, notamment une solution alcoolique ou hydro-alcoolique, ou une solution éthérée, d'un solvant organique apolaire, comme le n-hexane, le benzène, ou avantageusement une combinaison des deux.

3. Composition selon la revendication 1 ou 2, caractérisée en ce que les phases lamellaires lipidiques hydratées précitées contiennent en outre au moins en partie de l'acide kojique ou ses dérivés, notamment ses sels ou esters.

4. Composition selon la revendication 1, 2 ou 3, caractérisée en ce que les phases lamellaires lipidiques hydratées contiennent au moins en partie de l'hydroquinone seule ou en mélange avec de l'acide kojique ou ses dérivés, notamment ses sels ou esters.

5. Composition selon l'une des revendications 1 à 4, caractérisée en ce que l'extrait précité seul ou en mélange avec l'hydroquinone et/ou l'acide kojique ou ses dérivés, notamment ses sels ou esters, est introduit dans la phase lipidique des phases lamellaires lipidiques hydratées ou des liposomes.

6. Composition selon l'une des revendications 1 à 4, caractérisée en ce que l'extrait précité seul ou en mélange avec l'hydroquinone et/ou l'acide kojique ou ses dérivés, notamment ses sels ou esters, est introduit dans la phase aqueuse des phases lamellaires lipidiques hydratées ou des liposomes.

7. Composition à activité dépigmentante ou anti-inflammatoire cosmétique, caractérisée en ce qu'elle comprend une composition à base de phases lamellaires lipidiques hydratées ou de liposomes contenant au moins en partie un extrait de mûrier, notamment Morus Alba L. ou Morus Rubra, ou au moins une substance active isolée d'un tel extrait ou obtenue par synthèse chimique, en particulier une kuwanone.

8. Composition cosmétique, selon la revendication 7, caractérisée en ce que l'extrait précité est un extrait de feuilles, d'extrêmités de tiges, d'écorce de rejets ou d'écorce de racines, obtenu par une extraction par solvant, de préférence choisi parmi le groupe consistant d'un solvant polaire, notamment une solution alcoolique ou hydro-alcoolique, ou une solution éthérée, d'un solvant organique apolaire, comme le n-hexane, le benzène, ou avantageusement une combinaison des deux.

9. Composition cosmétique, selon l'une des revendications 7 ou 8, caractérisée en ce que les phases lamellaires lipidiques hydratées précitées contiennent en outre au moins en partie de l'acide kojique ou ses dérivés, notamment ses sels ou esters.

10. Composition cosmétique, selon l'une des revendications 7 à 9, caractérisée en ce que les phases lamellaires lipidiques hydratées précitées contiennent au moins en partie de l'hydroquinone seule ou en mélange avec de l'acide kojique ou des dérivés, notamment ses sels ou esters.

11. Composition cosmétique, selon l'une des revendications 7 à 10, caractérisée en ce que l'extrait précité seul ou en mélange avec l'hydroquinone et/ou l'acide kojique ou ses dérivés, notamment ses sels ou esters, est introduit dans la phase lipidique des phases lamellaires lipidiques hydratées ou des liposomes.

12. Composition cosmétique, selon l'une des revendications 7 à 10, caractérisée en ce que l'extrait précité seul ou en mélange avec l'hydroquinone et/ou l'acide kojique et ses dérivés, notamment ses sels ou esters, est introduit dans la phase aqueuse des phases lamellaires lipidiques hydratées ou des liposomes.

13. Composition cosmétique, selon l'une des revendications 7 à 12, caractérisée en ce que la proportion en poids d'extrait de mûrier est comprise entre 0,005 et 1 %, de préférence 0,005 et 0,1 %, et encore de préférence entre 0,05 et 0,1 %, en poids par rapport au poids total de la composition.

14. Composition cosmétique, selon l'une des revendications 7 à 13, caractérisée en ce que l'acide kojique ou ses dérivés, notamment ses sels ou esters, constitue de 0,5 à 4 %, de préférence 0,5 à 2 %, et encore de préférence environ 1 %, en poids par rapport au poids total de la composition.

15. Composition cosmétique, selon l'une des revendications 7 à 14, caractérisée en ce que l'hydroquinone constitue de 0,5 à 6 %, de préférence de 0,5 à 4 %, encore de préférence environ 2 %, en poids par rapport au poids total de la composition.

16. Composition cosmétique, caractérisée en ce qu'elle comprend une composition à base de phases lamellaires lipidiques hydratées ou de liposomes contenant au moins en partie un extrait d'extrémités de tiges, d'écorce de rejets ou d'écorce de racines de mûrier, notamment Morus Alba L. ou Morus Rubra, ou au moins une substance active isolée d'un tel extrait ou obtenue par synthèse chimique, en particulier une kuwanone.

17. Composition selon la revendication 16, caractérisée en ce qu'elle est telle que définie à l'une des revendications 2 à 6.

18. Composition selon la revendication 16 ou 17, caractérisée en ce que la proportion respective en poids d'extraits de mûrier, d'acide kojique ou ses dérivés ou d'hydroquinone est telle que définie à l'une des revendications 13, 14 ou 15.

19. Procédé de préparation d'une composition à activité dépigmentante ou anti-inflammatoire, cosmétique ou pharmaceutique, notamment dermatologique, caractérisé en ce qu'on incorpore au moins en partie dans lesdites phases lamellaires lipidiques hydratées ou lesdits liposomes, un extrait de mûrier, notamment Morus Alba L. ou Morus Rubra, ou au moins une substance active isolée d'un tel extrait ou obtenue par synthèse chimique, en parituclier une kuwanone.

20. Procédé selon la revendication 19, caractérisé en ce qu'on incorpore une concentration en poids d'extrait de mûrier seul comprise entre 0,005 et 1 %, de préférence 0,005 et 0,1 %, encore de préférence entre 0,05 et 0,1 % en poids par rapport au poids total de la composition, ou éventuellement en mélange avec l'hydroquinone à une concentration de 0,5 à 6 %, de préférence de 0,5 à 4 %, encore de préférence environ 2 % en poids par rapport au poids total de la composition, et/ou d'acide kojique et ses dérivés, notamment ses sels ou esters, à une concentration de 0,5 à 4%, de préférence 0,5 à 2 %, encore de préférence environ 1% en poids par rapport au poids total de la composition.

**Claims**

**Claims for the following Contracting States : AT, BE, CH, DE, GB, IT, LI, LU, NL, SE**

1. Composition based on hydrated lipidic lamellar phases or on liposomes, characterized in that said hydrated lipidic lamellar phases or the said liposomes contain at least part of an extract of mulberry, especially Morus Alba L. or Morus Rubra, or at least one active substance isolated from such an extract or obtained by chemical synthesis, in particular a kuwanone.

2. Composition according to claim 1, characterized in that the above-mentioned extract is an extract of leaves, ends of stems, bark of shoots or bark of roots, obtained by extraction with a solvent which is preferably selected from the group consisting of a polar solvent, especially an alcoholic or aqueous-alcoholic solution or an ether solution, an apolar organic solvent such as n-hexane or benzene, or advantageously a combination of both.

3. Composition according to claim 1 or 2, characterized in that the above-mentioned hydrated lipidic lamellar phases also contain, at least in part, kojic acid or derivatives thereof, especially salts or esters thereof.

4. Composition according to claim 1, 2 or 3, characterized in that the hydrated lipidic lamellar phases contain, at least in part, hydroquinone by itself or mixed with kojic acid or derivatives thereof, especially salts or esters thereof.

5. Composition according to one of claims 1 to 4, characterized in that the above-mentioned extract, by itself or mixed with hydroquinone and/or kojic acid or derivatives thereof, especially salts or esters thereof, is introduced into the lipidic phase of the hydrated lipidic lamellar phases or of the liposomes.

6. Composition according to one of claims 1 to 4, characterized in that the above-mentioned extract, by itself or mixed with hydroquinone and/or kojic acid or derivatives thereof, especially salts or esters thereof, is introduced into the aqueous phase of the hydrated lipidic lamellar phases or of the liposomes.

7. Cosmetic or pharmaceutical, especially dermatological composition, with skin-lightening or anti-inflammatory activity, characterized in that it comprises a composition based on hydrated lipidic lamellar phases or on liposomes, containing at least part of an extract of mulberry, especially Morus Alba L. or Morus Rubra, or at least one active substance isolated from such an extract or obtained by chemical synthesis, in particular a kuwanone.

8. Cosmetic or pharmaceutical, especially dermatological composition, according to claim 7, characterized in that the above-mentioned extract is an extract of leaves, ends of stems, bark of shoots or bark of roots, obtained by extraction with a solvent which is preferably selected from the group consisting of a polar solvent, especially an alcoholic or aqueous-alcoholic solution or an ether solution, an apolar organic solvent such as n-hexane or benzene, or advantageously a combination of both.

9. Cosmetic or pharmaceutical, especially dermatological composition, according to one of claims 7 or 8, characterized in that the above-mentioned hydrated lipidic lamellar phases also contain, at least in part, kojic acid or derivatives thereof, especially salts or esters thereof.

10. Cosmetic or pharmaceutical, especially dermatological composition, according to one of claims 7 to 9, characterized in that the above-mentioned hydrated lipidic lamellar phases contain, at least in part, hydroquinone by itself or mixed with kojic acid or derivatives thereof, especially salts or esters thereof.

11. Cosmetic or pharmaceutical, especially dermatological composition, according to one of claims 7 to 10, characterized in that the above-mentioned extract, by itself or mixed with hydroquinone and/or kojic acid or derivatives thereof, especially salts or esters thereof, is introduced into the lipidic phase of the hydrated lipidic lamellar phases or of the liposomes.

12. Cosmetic or pharmaceutical, especially dermatological composition, according to one of claims 7 to 10, characterized in that the above-mentioned extract, by itself or mixed with hydroquinone and/or kojic acid or derivatives thereof, especially salts or esters thereof, is introduced into the aqueous phase of the hydrated lipidic lamellar phases or of the liposomes.

13. Cosmetic or pharmaceutical, especially dermatological composition, according to one of claims 7 to 12, characterized in that the proportion by weight of mulberry extract is between 0.005 and 1%, preferably between 0.005 and 0.1% and more preferably between 0.05 and 0.1% by weight, relative to the total weight of the composition.

14. Cosmetic or pharmaceutical, especially dermatological composition, according to one of claims 7 to 13, characterized in that the kojic acid or derivatives thereof, especially salts or esters thereof, represents from 0.5 to 4%, preferably from 0.5 to 2% and more preferably about 1% by weight, relative to the total weight of the composition.

15. Cosmetic or pharmaceutical, especially dermatological composition, according to one of claims 7 to 14, characterized in that the hydroquinone represents from 0.5 to 6%, preferably from 0.5 to 4% and more preferably about 2% by weight, relative to the total weight of the composition.

16. Cosmetic composition, characterized in that it comprises a composition based on hydrated lipidic lamellar phases or on liposomes containing at least part of an extract of ends of stems, bark of shoots or bark of roots of mulberry, especially Morus Alba L. or Morus Rubra, or at least one active substance isolated from such an extract or obtained by chemical synthesis, in particular a kuwanone.

17. Composition according to claim 16, characterized in that it is as defined in one of claims 2 to 6.

18. Composition according to claim 16 or 17, characterized in that the respective proportion by weight of mulberry extract, of kojic acid or derivatives thereof, or of hydroquinone is as defined in one of claims 13, 14 or 15.

**Claims for the following Contracting State : ES**

1. Process for the preparation of a composition based on hydrated lipidic lamellar phases or on liposomes, characterized in that at least part of an extract of mulberry, especially Morus Alba L. or Morus Rubra, or at least one active substance isolated from such an extract or obtained by chemical synthesis, in particular a kuwanone, is incorporated into said hydrated lipidic lamellar phases or the said liposomes.

2. Preparation process according to claim 1, characterized in that an extract of leaves, ends of stems, bark of shoots or bark of roots, obtained by extraction with a solvent, is prepared, which is preferably selected from the group consisting of a polar solvent, especially an alcoholic or aqueous-alcoholic solution or an ether solution, an apolar organic solvent such as n-hexane or benzene, or advantageously a combination of both.

3. Process according to claim 1 or 2, characterized in that the above-mentioned hydrated lipidic lamellar phases also contain, et least in part, kojic acid or derivatives thereof, especially salts or esters thereof.

4. Process according to claim 1, 2 or 3, characterized in that the hydrated lipidic lamellar phases contain, at least in part, hydroquinone by itself or mixed with kojic acid or derivatives thereof, especially salts or esters thereof.

5. Process according to claims 1 to 4, characterized in that the above-mentioned extract, by itself or mixed with hydroquinone and/or kojic acid or derivetives thereof, especially salts or esters thereof, is introduced into the lipidic phase of the hydrated lipidic lamellar phases or of the liposomes.

6. Process according to one of claims 1 to 4, characterized in that the above-mentioned extract, by itself or mixed with hydroquinone and/or kojic acid or derivatives thereof, especially salts or esters thereof, is introduced into the aqueous phase of the hydrated lipidic lamellar phases or of the liposomes.

7. Process for the preparation of a cosmetic or pharmaceutical, especially dermatological composition, with skin-lightening or anti-inflammatory activity, characterized in that at least part of an extract of mulberry, especially Morus Alba L. or Morus Rubra, or at least one active substance isolated from such an extract or obtained by chemical synthesis, in particular a kuwanone, is incorporated into said hydrated lipidic lamellar phases or on liposomes.

8. Process according to claim 7, characterized in that the above-mentioned extract is an extract of leaves, ends of stems, bark of shoots or bark of roots, obtained by extraction with a solvent which is preferably selected from the group consisting of a polar solvent, especially an alcoholic or aqueous-alcoholic solution or an ether solution, an apolar organic solvent such as n-hexane or benzene, or advantageously a combination of both.

9. Process according to one of claims 7 or 8, characterized in that the above-mentioned hydrated lipidic lamellar phases also contain, at least in part, kojic acid or derivatives thereof, especially salts or esters thereof.

10. Process according to one of claims 7 to 9, characterized in that the above-mentioned hydrated lipidic lamellar phases contain, at least in part, hydroquinone by itself or mixed with kojic acid or derivatives thereof, especially salts or esters thereof.

11. Process according to one of claims 7 to 10, characterized in that the above-mentioned extract, by itself or mixed with hydroquinone and/or kojic acid or derivatives thereof, especially salts or esters thereof, is introduced into the lipidic phase of the hydrated lipidic lamellar phases or of the liposomes.

12. Process according to one of claims 7 to 10, characterized in that the above-mentioned extract, by itself or mixed with hydroquinone and/or kojic acid or derivatives thereof, especially salts or esters thereof, is introduced into the aqueous phase of the hydrated lipidic lamellar phases or of the liposomes.

13. Process according to one of claims 7 to 12, characterized in that between 0.005 and 1%, preferably between 0.005 and 0.1% and more preferably between 0.05 and 0.1% by weight of mulberrry extract is incorporated, relative to the total weight of the composition.

14. Process according to one of claims 7 to 13, characterized in that from 0.5 to 4%, preferably from 0.5 to 2% and more preferably about 1% by weight of kojic acid or derivatives thereof, especially salts or esters thereof is incorporated, relative to the total weight of the composition.

15. Process according to one of claims 7 to 14, characterized in that from 0.5 to 6%, preferably from 0.5 to 4% and more preferably about 2% by weight of hydroquinone is incorporated, relative to the total weight of the composition.

16. Process for the preparation of a cosmetic composition based on hydrated lipidic lamellar phases or on liposomes, characterized in that at least part of an extract of ends of stems, bark of shoots or bark of roots of mulberry, especially Morus Alba L. or Morus Rubra, or at least one active substance isolated from such an extract or obtained by chemical synthesis, in particular a kuwanone is incorporated into said hydrated lipidic lamellar phases or said liposomes.

17. Process according to claim 16, characterized in that the procedure used is as described in any one of claims 2 to 6.

18. Process according to claim 16 or 17, characterized in that a concentration by weight of mulberry extract, of hydroquinone and/or of kojic acid or derivatives thereof, respectively, is incorporated as defined in one of claims 13, 14 or 15.

**Claims for the following Contracting State : GR**

1. Composition based on hydrated lipidic lamellar phases or on liposomes, characterized in that said hydrated lipidic lamellar phases or the said liposomes contain at least part of an extract of mulberry, especially Morus Alba L. or Morus Rubra, or at least one active substance isolated from such an extract or obtained by chemical synthesis, in particular a kuwanone.

2. Composition according to claim 1, characterized in that the above-mentioned extract is an extract of leaves, ends of stems, bark of shoots or bark of roots, obtained by extraction with a solvent which is preferably selected from the group consisting of a polar solvent, especially an alcoholic or aqueous-alcoholic solution or an ether solution, an apolar organic solvent such as n-hexane or benzene, or advantageously a combination of both.

3. Composition according to claim 1 or 2, characterized in that the above-mentioned hydrated lipidic lamellar phases also contain, at least in part, kojic acid or derivatives thereof, especially salts or esters thereof.

4. Composition according to claim 1, 2 or 3, characterized in that the hydrated lipidic lamellar phases contain, at least in part, hydroquinone by itself or mixed with kojic acid or derivatives thereof, especially salts or esters thereof.

5. Composition according to one of claims 1 to 4, characterized in that the above-mentioned extract, by itself or mixed with hydroquinone and/or kojic acid or derivatives thereof, especially salts or esters thereof, is introduced into the lipidic phase of the hydrated lipidic lamellar phases or of the liposomes.

6. Composition according to one of claims 1 to 4, characterized in that the above-mentioned extract, by itself or mixed with hydroquinone and/or kojic acid or derivatives thereof, especially salts or esters thereof, is introduced into the aqueous phase of the hydrated lipidic lamellar phases or of the liposomes.

7. Cosmetic composition with skin-lightening or anti-inflammatory activity, characterized in that it comprises a composition based on hydrated lipidic lamellar phases or on liposomes, containing at least part of an extract of mulberry, especially Morus Alba L. or Morus Rubra, or at least one active substance isolated from such an extract or obtained by chemical synthesis, in particular a kuwanone.

8. Cosmetic composition according to claim 7, characterized in that the above-mentioned extract is an extract of leaves, ends of stems, bark of shoots or bark of roots, obtained by extraction with a solvent which is preferably selected from the group consisting of a polar solvent, especially an alcoholic or aqueous-alcoholic solution or an ether solution, an apolar organic solvent such as n-hexane or benzene, or advantageously a combination of both.

9. Cosmetic composition according to one of claims 7 or 8, characterized in that the above-mentioned hydrated lipidic lamellar phases also contain, at least in part, kojic acid or derivatives thereof, especially salts or esters thereof.

10. Cosmetic composition according to one of claims 7 to 9, characterized in that the above-mentioned hydrated lipidic lamellar phases contain, at least in part, hydroquinone by itself or mixed with kojic acid or derivatives thereof, especially salts or esters thereof.

11. Cosmetic composition according to one of claims 7 to 10, characterized in that the above-mentioned extract, by itself or mixed with hydroquinone and/or kojic acid or derivatives thereof, especially salts or esters thereof, is introduced into the lipidic phase of the hydrated lipidic lamellar phases or of the liposomes.

12. Cosmetic composition according to one of claims 7 to 10, characterized in that the above-mentioned extract, by itself or mixed with hydroquinone and/or kojic acid or derivatives thereof, especially salts or eaters thereof, is introduced into the aqueous phase of the hydrated lipidic lamellar phases or of the liposomes.

13. Cosmetic composition according to one of claims 7 to 12, characterized in that the proportion by weight of mulberry extract is between 0.005 and 1%, preferably between 0.005 and 0.1% and more preferably between 0.05 and 0.1% by weight, relative to the total weight of the composition.

14. Cosmetic composition according to one of claims 7 to 13, characterized in that the kojic acid or derivatives thereof, especially salts or esters thereof, represents from 0.5 to 4%, preferably from 0.5 to 2% and more preferably about 1% by weight, relative to the total weight of the composition.

15. Cosmetic composition according to one of claims 7 to 14, characterized in that the hydroquinone represents from 0.5 to 6%, preferably from 0.5 to 4% and more preferably about 2% by weight, relative to the total weight of the composition.

16. Cosmetic composition, characterized in that it comprises a composition based on hydrated lipidic lamellar phases or on liposomes containing at least part of an extract of ends of stems, bark of shoots or bark of roots of mulberry, especially Morus Alba L. or Morus Rubra, or at least one active substance isolated from such an extract or obtained by chemical synthesis, in particular a kuwanone.

17. Composition according to claim 16, characterized in that it is as defined in one of claims 2 to 6.

18. Composition according to claim 16 or 17, characterized in that the respective proportion by weight of mulberry extract, of kojic acid or derivatives thereof, or of hydroquinone is as defined in one of claims 13, 14 or 15.

19. Process for the preparation of a cosmetic or pharmaceutical, especially dermatological composition with skin-lightening or anti-inflammatory activity, characterized in that at least part of an extract of mulberry, especially Morus Alba L. or Morus Rubra, or at least one active substance isolated from such an extract or obtained by chemical synthesis, in particular a kuwanone is incorporated into said hydrated lipidic lamellar phases or said liposomes.

20. Process according to claim 19, characterized in that a concentration by weight of an extract of mulberry by itself comprised between 0.005 and 1%, preferably between 0.005 and 0.1% and more preferably between 0.05 and 0.1% by weight is incorporated, relative to the total weight of the composition, or optionally in a mixture with the hydroquinone at a concentration from 0.5 to 6%, preferably from 0.5 to 4% and more preferably about 2% by weight relative to the total weight of the composition, and/or of kojic acid or derivatives thereof, especially salts or esters thereof at a concentration from 0.5 to 4%, preferably from 0.5 to 2% and more preferably about 1% by weight relative to the total weight of the composition.

**Patentansprüche**

**Patentansprüche für folgende Vertragsstaaten : AT, BE, CH, DE, GB, IT, LI, LU, NL, SE**

1. Zusammensetzung auf Basis von wasserhaltigen lamellaren Lipidphasen oder von Liposomen, dadurch gekennzeichnet, daß die wasserhaltigen lamellaren Lipidphasen oder die Liposome zumindest teilweise einen Extrakt des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, enthalten.

2. Zusammensetzung nach Anspruch 1, dadurch gekennzeichnet, daß der Extrakt aus Blättern, Stengelenden, Rinde von Trieben oder Wurzeln ist, der durch Extraktion mit einem Lösungsmittel, vorzugsweise ausgewählt aus polaren Lösungsmitteln, insbesondere alkoholischen oder wasserhaltigen alkoholischen Lösungen oder Etherlösungen, organischen apolaren Lösungsmitteln, wie n-Hexan oder Benzol, oder vorteilhafterweise einer Kombination von beiden, erhalten ist.

3. Zusammensetzung nach Anspruch 1 oder 2, dadurch gekennzeichnet, daß die wasserhaltigen lamella-

EP 0 296 923 B1

ren Lipidphasen außerdem mindestens teilweise Kojisäure oder ihre Derivate, insbesondere ihre Salze oder Ester, enthalten.

4. Zusammensetzung nach einem der Ansprüche 1 bis 3, dadurch gekennzeichnet, daß die wasserhaltigen lamellaren Lipidphasen mindestens teilweise Hydrochinon allein oder in Mischung mit Kojisäure oder ihren Derivaten, insbesondere ihren Salzen oder Estern, enthalten.

5. Zusammensetzung nach einem der Ansprüche 1 bis 4, dadurch gekennzeichnet, daß der Extrakt allein oder in Mischung mit Hydrochinon und/oder Kojisäure oder ihren Derivaten, insbesondere ihren Salzen oder Estern, in die Lipidphase der wasserhaltigen lamellaren Lipidphasen oder der Liposome eingeführt ist.

6. Zusammensetzung nach einem der Ansprüche 1 bis 4, dadurch gekennzeichnet, daß der Extrakt allein oder in Mischung mit Hydrochinon und/oder Kojisäure oder ihren Derivaten, insbesondere ihren Salzen oder Estern, in die Wasserphase der wasserhaltigen lamellaren Lipidphasen oder der Liposome eingeführt ist.

7. Kosmetische oder pharmazeutische, insbesondere dermatologische Zusammensetzung mit depigmentierender oder entzündungshemmender Wirkung, dadurch gekennzeichnet, daß sie eine Zusammensetzung auf Basis von wasserhaltigen lamellaren Lipidphasen oder von Liposomen aufweist, die zumindest teilweise einen Extrakt des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, enthalten.

8. Kosmetische oder pharmazeutische, insbesondere dermatologische Zusammensetzung nach Anspruch 7, dadurch gekennzeichnet, daß der Extrakt ein Extrakt aus Blättern, Stengelenden, Rinde von Trieben oder Wurzeln ist, der durch Extraktion mit einem Lösungsmittel, vorzugsweise ausgewählt aus polaren Lösungsmitteln, insbesondere alkoholischen oder wasserhaltigen alkoholischen Lösungen oder Etherlösungen, organischen apolaren Lösungsmitteln, wie n-Hexan oder Benzol, oder vorteilhafterweise einer Kombination von beiden, erhalten ist.

9. Kosmetische oder pharmazeutische, insbesondere dermatologische Zusammensetzung nach Anspruch 7 oder 8, dadurch gekennzeichnet, daß die wasserhaltigen lamellaren Lipidphasen außerdem mindestens teilweise Kojisäure oder ihre Derivate, insbesondere ihre Salze oder Ester, enthalten.

10. Kosmetische oder pharmazeutische, insbesondere dermatologische Zusammensetzung nach einem der Ansprüche 7 bis 9, dadurch gekennzeichnet, daß die wasserhaltigen lamellaren Lipidphasen mindestens teilweise Hydrochinon allein oder in Mischung mit Kojisäure oder ihren Derivaten, insbesondere ihren Salzen oder Estern, enthalten.

11. Kosmetische oder pharmazeutische, insbesondere dermatologische Zusammensetzung nach einem der Ansprüche 7 bis 10, dadurch gekennzeichnet, daß der Extrakt allein oder in Mischung mit Hydrochinon und/oder Kojisäure oder ihren Derivaten, insbesondere ihren Salzen oder Estern, in die Lipidphase der wasserhaltigen lamellaren Lipidphasen oder der Liposome eingeführt ist.

12. Kosmetische oder pharmazeutische, insbesondere dermatologische Zusammensetzung nach einem der Ansprüche 7 bis 10, dadurch gekennzeichnet, daß der Extrakt allein oder in Mischung mit Hydrochinon und/oder Kojisäure und ihren Derivaten, insbesondere ihren Salzen oder Estern, in die Wasserphase der wasserhaltigen lamellaren Lipidphasen oder der Liposome eingeführt ist.

13. Kosmetische oder pharmazeutische, insbesondere dermatologische Zusammensetzung nach einem der Ansprüche 7 bis 12, dadurch gekennzeichnet, daß der Gewichtsanteil an Maulbeerbaumextrakt 0,005 bis 1 %, vorzugsweise 0,005 bis 0,1 %, insbesondere 0,05 bis 0,1 %, bezogen auf das Gesamtgewicht der Zusammensetzung, beträgt.

14. Kosmetische oder pharmazeutische, insbesondere dermatologische Zusammensetzung nach einem der Ansprüche 7 bis 13, dadurch gekennzeichnet, daß die Kojisäure oder ihre Derivate, insbesondere ihre Salze oder Ester, 0,5 bis 4 %, vorzugsweise 0,5 bis 2 %, insbesondere etwa 1 %, bezogen auf das

19

Gesamtgewicht der Zusammensetzung, ausmacht.

**15.** Kosmetische oder pharmazeutische, insbesondere dermatologische Zusammensetzung nach einem der Ansprüche 7 bis 14, dadurch gekennzeichnet, daß das Hydrochinon 0,5 bis 6 %, vorzugsweise 0,5 bis 4 %, insbesondere etwa 2 %, bezogen auf das Gesamtgewicht der Zusammensetzung, ausmacht.

**16.** Kosmetische Zusammensetzung, dadurch gekennzeichnet, daß sie eine Zusammensetzung auf Basis von wasserhaltigen lamellaren Lipidphasen oder von Liposomen, die zumindest teilweise einen Extrakt von Stengelenden, Triebrinde oder Wurzelrinde des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, enthalten, umfaßt.

**17.** Zusammensetzung nach Anspruch 16, dadurch gekennzeichnet, daß sie wie in einem der Ansprüche 2 bis 6 definiert ist.

**18.** Zusammensetzung nach Anspruch 16 oder 17, dadurch gekennzeichnet, daß das jeweilige Gewichtsverhältnis von Maulbeerbaumextrakten, Kojisäure oder ihren Derivaten oder von Hydrochinon wie in einem der Ansprüche 13, 14 oder 15 definiert ist.

**Patentansprüche für folgenden Vertragsstaat : ES**

**1.** Verfahren zur Herstellung einer Zusammensetzung auf Basis von wasserhaltigen lamellaren Lipidphasen oder von Liposomen, dadurch gekennzeichnet, daß man zumindest teilweise in die wasserhaltigen lamellaren Lipidphasen oder in die Liposome einen Extrakt des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, einarbeitet.

**2.** Herstellungsverfahren nach Anspruch 1, dadurch gekennzeichnet, daß man einen Extrakt aus Blättern, Stengelenden, Rinde von Trieben oder Wurzeln bereitet, der durch Extraktion mit einem Lösungsmittel, vorzugsweise ausgewählt aus polaren Lösungsmitteln, insbesondere alkoholischen oder wasserhaltigen alkoholischen Lösungen oder Etherlösungen, organischen apolaren Lösungsmitteln, wie n-Hexan oder Benzol, oder vorteilhafterweise einer Kombination von beiden, erhalten wird.

**3.** Verfahren nach Anspruch 1 oder 2, dadurch gekennzeichnet, daß die wasserhaltigen lamellaren Lipidphasen außerdem mindestens teilweise Kojisäure oder ihre Derivate, insbesondere ihre Salze oder Ester, enthalten.

**4.** Verfahren nach Anspruch 1, 2 oder 3, dadurch gekennzeichnet, daß die wasserhaltigen lamellaren Lipidphasen mindestens teilweise Hydrochinon allein oder in Mischung mit Kojisäure oder ihren Derivaten, insbesondere ihren Salzen oder Estern, enthalten.

**5.** Verfahren nach den Ansprüchen 1 bis 4, dadurch gekennzeichnet, daß der Extrakt allein oder in Mischung mit Hydrochinon und/oder Kojisäure oder ihren Derivaten, insbesondere ihren Salzen oder Estern, in die Lipidphase der wasserhaltigen lamellaren Lipidphasen oder der Liposome eingeführt wird.

**6.** Verfahren nach einem der Ansprüche 1 bis 4, dadurch gekennzeichnet, daß der Extrakt allein oder in Mischung mit Hydrochinon und/oder Kojisäure oder ihren Derivaten, insbesondere ihren Salzen oder Estern, in die Wasserphase der wasserhaltigen lamellaren Lipidphasen oder der Liposome eingeführt wird.

**7.** Verfahren zur Herstellung einer kosmetischen oder pharmazeutischen, insbesondere dermatologischen Zusammensetzung mit depigmentierender oder entzündungshemmender Wirkung, dadurch gekennzeichnet, daß zumindest teilweise in die wasserhaltigen lamellaren Lipidphasen oder Liposome ein Extrakt des Maulbeerbaums, insbesondere von Morus Alba L. oder Morus Rubra, oder mindestens ein aus einem solchen Extrakt isolierter oder durch chemische Synthese erhaltener Wirkstoff, insbesondere ein Kuwanon, eingearbeitet wird.

**8.** Verfahren nach Anspruch 7, dadurch gekennzeichnet, daß der Extrakt ein Extrakt aus Blättern, Stengelenden, Rinde von Trieben oder Wurzeln ist, der durch Extraktion mit einem Lösungsmittel, vorzugsweise ausgewählt aus polaren Lösungsmitteln, insbesondere alkoholischen oder wasserhaltigen alkoholischen Lösungen oder Etherlösungen, organischen apolaren Lösungsmitteln, wie n-Hexan oder Benzol, oder vorteilhafterweise einer Kombination von beiden, erhalten wird.

9. Verfahren nach einem der Ansprüche 7 oder 8, dadurch gekennzeichnet, daß die wasserhaltigen lamellaren Lipidphasen außerdem mindestens teilweise Kojisäure oder ihre Derivate, insbesondere ihre Salze oder Ester, enthalten.

10. Verfahren nach einem der Ansprüche 7 bis 9, dadurch gekennzeichnet, daß die wasserhaltigen lamellaren Lipidphasen mindestens teilweise Hydrochinon allein oder in Mischung mit Kojisäure oder ihren Derivaten, insbesondere ihren Salzen oder Estern, enthalten.

11. Verfahren nach einem der Ansprüche 7 bis 10, dadurch gekennzeichnet, daß der Extrakt allein oder in Mischung mit Hydrochinon und/oder Kojisäure oder ihren Derivaten, insbesondere ihren Salzen oder Estern, in die Lipidphase der wasserhaltigen lamellaren Lipidphasen oder der Liposome eingeführt wird.

12. Verfahren nach einem der Ansprüche 7 bis 10, dadurch gekennzeichnet, daß der Extrakt allein oder in Mischung mit Hydrochinon und/oder Kojisäure und ihren Derivaten, insbesondere ihren Salzen oder Estern, in die Wasserphase der wasserhaltigen lamellaren Lipidphasen oder der Liposome eingeführt wird.

13. Verfahren nach einem der Ansprüche 7 bis 12, dadurch gekennzeichnet, daß 0,005 bis 1 %, vorzugsweise 0,005 bis 0,1 %, insbesondere 0,05 bis 0,1 %, an Maulbeerbaumextrakt, bezogen auf das Gesamtgewicht der Zusammensetzung, eingearbeitet werden.

14. Verfahren nach einem der Ansprüche 7 bis 13, dadurch gekennzeichnet, daß 0,5 bis 4 %, vorzugsweise 0,5 bis 2 %, insbesondere etwa 1 %, Kojisäure oder deren Derivate, insbesondere deren Salze oder Ester, bezogen auf das Gesamtgewicht der Zusammensetzung, eingearbeitet werden.

15. Verfahren nach einem der Ansprüche 7 bis 14, dadurch gekennzeichnet, daß 0,5 bis 6 %, vorzugsweise 0,5 bis 4 %, insbesondere etwa 2 %, Hydrochinon, bezogen auf das Gesamtgewicht der Zusammensetzung, eingearbeitet werden.

16. Verfahren zur Herstellung einer kosmetischen Zusammensetzung auf Basis von wasserhaltigen lamellaren Lipidphasen oder von Liposomen, dadurch gekennzeichnet, daß man zumindest teilweise in die wasserhaltigen lamellaren Lipidphasen oder Liposome einen Extrakt von Stengelenden, Triebrinde oder Wurzelrinde des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, einarbeitet.

17. Verfahren nach Anspruch 16, dadurch gekennzeichnet, daß, wie in einem der Ansprüche 2 bis 6 beschrieben, vorgegangen wird.

18. Verfahren nach Anspruch 16 oder 17, dadurch gekennzeichnet, daß eine jeweilige Gewichtskonzentration von Maulbeerbaumextrakten, Hydrochinon und/oder Kojisäure oder deren Derivaten, wie in einem der Ansprüche 13, 14 oder 15 definiert, eingearbeitet wird.

**Patentansprüche für folgenden Vertragsstaat : GR**

1. Zusammensetzung auf Basis von wasserhaltigen lamellaren Lipidphasen oder von Liposomen, dadurch gekennzeichnet, daß die wasserhaltigen lamellaren Lipidphasen oder die Liposome zumindest teilweise einen Extrakt des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, enthalten.

2. Zusammensetzung nach Anspruch 1, dadurch gekennzeichnet, daß der Extrakt ein Extrakt aus Blättern, Stengelenden, Rinde von Trieben oder Wurzeln ist, der durch Extraktion mit einem Lösungsmittel, vorzugsweise ausgewählt aus polaren Lösungsmitteln, insbesondere alkoholischen oder wasserhaltigen alkoholischen Lösungen oder Etherlösungen, organischen apolaren Lösungsmitteln, wie n-Hexan oder Benzol, oder vorteilhafterweise einer Kombination von beiden, erhalten ist.

3. Zusammensetzung nach Anspruch 1 oder 2, dadurch gekennzeichnet, daß die wasserhaltigen lamellaren Lipidphasen außerdem mindestens teilweise Kojisäure oder ihre Derivate, insbesondere ihre Salze oder Ester, enthalten.

4. Zusammensetzung nach einem der Ansprüche 1, 2 oder 3, dadurch gekennzeichnet, daß die wasserhaltigen lamellaren Lipidphasen mindestens teilweise Hydrochinon allein oder in Mischung mit Kojisäure oder ihren Derivaten, insbesondere ihren Salzen oder Estern, enthalten.

5. Zusammensetzung nach einem der Ansprüche 1 bis 4, dadurch gekennzeichnet, daß der Extrakt allein oder in Mischung mit Hydrochinon und/oder Kojisäure oder ihren Derivaten, insbesondere ihren Salzen oder Estern, in die Lipidphase der wasserhaltigen lamellaren Lipidphasen oder der Liposome eingeführt ist.

6. Zusammensetzung nach einem der Ansprüche 1 bis 4, dadurch gekennzeichnet, daß der Extrakt allein oder in Mischung mit Hydrochinon und/oder Kojisäure oder ihren Derivaten, insbesondere ihren Salzen oder Estern, in die Wasserphase der wasserhaltigen lamellaren Lipidphasen oder der Liposome eingeführt ist.

7. Kosmetische Zusammensetzung mit depigmentierender oder entzündungshemmender Wirkung, dadurch gekennzeichnet, daß sie eine Zusammensetzung auf Basis von wasserhaltigen lamellaren Lipidphasen oder von Liposomen aufweist, die zumindest teilweise einen Extrakt des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, enthalten.

8. Kosmetische Zusammensetzung nach Anspruch 7, dadurch gekennzeichnet, daß ein Extrakt aus Blättern, Stengelenden, Rinde von Trieben oder Wurzeln ist, der durch Extraktion mit einem Lösungsmittel, vorzugsweise ausgewählt aus polaren Lösungsmitteln, insbesondere alkoholischen oder wasserhaltigen alkoholischen Lösungen oder Etherlösungen, organischen apolaren Lösungsmitteln, wie n-Hexan oder Benzol, oder vorteilhafterweise einer Kombination von beiden, erhalten ist.

9. Kosmetische Zusammensetzung nach Anspruch 7 oder 8, dadurch gekennzeichnet, daß die wasserhaltigen lamellaren Lipidphasen außerdem mindestens teilweise Kojisäure oder ihre Derivate, insbesondere ihre Salze oder Ester, enthalten.

10. Kosmetische Zusammensetzung nach einem der Ansprüche 7 bis 9, dadurch gekennzeichnet, daß die wasserhaltigen lamellaren Lipidphasen mindestens teilweise Hydrochinon allein oder in Mischung mit Kojisäure oder ihren Derivaten, insbesondere ihren Salzen oder Estern, enthalten.

11. Kosmetische Zusammensetzung nach einem der Ansprüche 7 bis 10, dadurch gekennzeichnet, daß der Extrakt allein oder in Mischung mit Hydrochinon und/oder Kojisäure oder ihren Derivaten, insbesondere ihren Salzen oder Estern, in die Lipidphase der wasserhaltigen lamellaren Lipidphasen oder der Liposome eingeführt ist.

12. Kosmetische Zusammensetzung nach einem der Ansprüche 7 bis 10, dadurch gekennzeichnet, daß der Extrakt allein oder in Mischung mit Hydrochinon und/oder Kojisäure und ihren Derivaten, insbesondere ihren Salzen oder Estern, in die Wasserphase der wasserhaltigen lamellaren Lipidphasen oder der Liposome eingeführt ist.

13. Kosmetische Zusammensetzung nach einem der Ansprüche 7 bis 12, dadurch gekennzeichnet, daß der Gewichtsanteil an Maulbeerbaumextrakt 0,005 bis 1 %, vorzugsweise 0,005 bis 0,1 %, insbesondere 0,05 bis 0,1 %, bezogen auf das Gesamtgewicht der Zusammensetzung, beträgt.

14. Kosmetische Zusammensetzung nach einem der Ansprüche 7 bis 13, dadurch gekennzeichnet, daß die Kojisäure oder ihre Derivate, insbesondere ihre Salze oder Ester, 0,5 bis 4 %, vorzugsweise 0,5 bis 2

EP 0 296 923 B1

%, insbesondere etwa 1 %, bezogen auf das Gesamtgewicht der Zusammensetzung, ausmachen.

**15.** Kosmetische Zusammensetzung nach einem der Ansprüche 7 bis 14, dadurch gekennzeichnet, daß das Hydrochinon 0,5 bis 6 %, vorzugsweise 0,5 bis 4 %, insbesondere etwa 2 %, bezogen auf das Gesamtgewicht der Zusammensetzung, ausmacht.

**16.** Kosmetische Zusammensetzung, dadurch gekennzeichnet, daß sie eine Zusammensetzung auf Basis von wasserhaltigen lamellaren Lipidphasen oder von Liposomen, die zumindest teilweise einen Extrakt von Stengelenden, Triebrinde oder Wurzelrinde des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, enthalten, umfaßt.

**17.** Zusammensetzung nach Anspruch 16, dadurch gekennzeichnet, daß sie wie in einem der Ansprüche 2 bis 6 definiert ist.

**18.** Zusammensetzung nach Anspruch 16 oder 17, dadurch gekennzeichnet, daß das jeweilige Gewichtsverhältnis von Maulbeerbaumextrakten, Kojisäure oder ihren Derivaten oder von Hydrochinon wie in einem der Ansprüche 13, 14 oder 15 definiert ist.

**19.** Verfahren zur Herstellung einer kosmetischen oder pharmazeutischen, insbesondere dermatologischen Zusammensetzung mit depigmentierender oder entzündungshemmender Wirkung, dadurch gekennzeichnet, daß man zumindest teilweise in die wasserhaltigen lamellaren Lipidphasen oder in die Liposome einen Extrakt des Maulbeerbaums, insbesondere von Morus Alba L. oder Morus Rubra, oder mindestens einen aus einem solchen Extrakt isolierten oder durch chemische Synthese erhaltenen Wirkstoff, insbesondere ein Kuwanon, einarbeitet.

**20.** Verfahren nach Anspruch 19, dadurch gekennzeichnet, daß man eine Gewichtskonzentration an Maulbeerbaumextrakt allein von 0,005 bis 1 %, vorzugsweise 0,005 bis 0,1 %, insbesondere 0,05 bis 0,1 %, bezogen auf das Gesamtgewicht der Zusammensetzung, oder gegebenenfalls in Mischung mit Hydrochinon in einer Konzentration von 0,5 bis 6 %, vorzugsweise 0,5 bis 4 %, insbesondere etwa 2 %, bezogen auf das Gesamtgewicht der Zusammensetzung, und/oder mit Kojisäure oder deren Derivate, insbesondere deren Salze oder Ester, in einer Konzentration von 0,5 bis 4 %, vorzugsweise 0,5 bis 2 %, insbesondere etwa 1 %, bezogen auf das Gesamtgewicht der Zusammensetzung, einarbeitet.